# FASCICULE DE BREVET EUROPEEN

(11) **EP 0 651 986 B1**
(45) Date de publication et mention de la délivrance du brevet: **09.09.1998**
(21) Numéro de dépôt: 94402441.3
(22) Date de dépôt: 28.10.1994
(51) Int. Cl.: A61F 13/15

(54) **Serviette périodique à incurvation réglable**
Monatsbinde mit verstellbarer Wölbung
Sanitary towel with adjustable camber

(30) Priorité: 10.11.1993 FR 9313415
(43) Date de publication de la demande: 10.05.1995
(73) Titulaire: FORT JAMES FRANCE, 68320 Kunheim (FR)
(72) Inventeur: Lefebvre du Grosriez, Carol, F-78600 Maisons Lafitte (FR)
(74) Mandataire: David, Daniel

(56) Documents cités:
- EP-A- 0 426 235
- WO-A-93/06805
- GB-A- 2 001 236
- GB-A- 2 233 235
- US-A- 5 127 911

## Description

La présente invention concerne un nouveau dispositif formant serviette périodique à incurvation réglable.

On connaît déjà de multiples dispositifs formant serviettes périodiques ou similaires.

D'une façon générale, on a cherché à accroître la capacité d'absorption des produits utilisés de façon à diminuer l'épaisseur, augmentant ainsi également le confort. De nombreuses recherches ont également été faites pour augmenter l'étanchéité et en particulier l'étanchéité latérale.

Ainsi, pour améliorer l'étanchéité dans le cas de serviettes comprenant un élément tampon biconcave à l'intérieur d'une enveloppe perméable aux fluides, on a proposé dans le brevet EP-A-0267059 de sceller ladite enveloppe au niveau de la zone biconcave de façon à réaliser, grâce à ce scellage latéral, des volets soudés, ce scellage ayant en particulier, du fait de l'épaisseur et de la forme du tampon absorbant, pour fonction de tendre le voile supérieur de la zone médiane.

On a également cherché à améliorer la protection du linge de corps en prévoyant des volets latéraux destinés à être rabattus autour dudit linge. On connaît de nombreux brevets concernant des serviettes périodiques munies de panneaux latéraux qui sont formés soit intégralement avec l'élément central, soit séparément et fixés sur lui.

Ainsi le brevet US 4285343 décrit des serviettes périodiques comprenant une partie absorbante centrale et des panneaux latéraux qui peuvent être soit intégrés, soit formés séparément et attachés ensuite et dont la ligne de jonction avec l'élément central doit être flexible de façon que l'on puisse replier lesdits panneaux au-dessus de la partie centrale lors de l'emballage ou au-dessous lors de l'usage du dispositif.

On a cherché à améliorer le confort des serviettes périodiques minces au niveau de l'entrejambe et à améliorer la protection en développant de nouvelles serviettes minces présentant sur une partie au moins de la zone d'entrejambes un bourrelet destiné à améliorer leur confort. De telles serviettes périodiques sont décrites dans la demande de brevet européen EP 0520884. De telles serviettes peuvent éventuellement être munies de volets latéraux.

Le brevet EP 0331018 décrit des serviettes périodiques destinées à assurer une meilleure protection des sous-vêtements, et comprennent sur leur zone d'entrejambes des volets latéraux élastiques comprenant des parties concaves vers l'extérieur.

Toujours avec le même souci d'améliorer le confort et la capacité de protection, le brevet européen EP 0155515 prévoit de munir les bords latéraux des serviettes, sur 10 à 30 % de leur longueur, de moyens élastiques permettant de faire prendre à la couche absorbante un profil en forme de coupe destiné à servir de réservoir au flux menstruel.

Le brevet français FR 2681242 décrit une serviette périodique dont l'enveloppe présente des découpes sinusoïdales formant des volets latéraux lorsque la serviette est dépliée ainsi que des volets à ses extrémités.

Cette découpe particulière, destinée à créer une sorte de coquille prévue pour s'adapter à l'anatomie de l'utilisatrice, présente l'inconvénient d'être de réalisation complexe.

Par ailleurs, il a été décrit dans le brevet EP 0269878 des serviettes périodiques munies de cordonnets le long de leurs bords latéraux, ces cordonnets étant destinés à assurer l'enroulement de la serviette sur elle-même avant utilisation pour son stockage ou après son utilisation avant d'être jetée.

La Demanderesse, au cours de ses recherches pour améliorer à la fois le confort et la capacité de protection des serviettes périodiques, a maintenant découvert un nouveau type de serviettes périodiques munies de volets latéraux dont on peut adapter à volonté l'incurvation de façon à mieux conformer ces serviettes aux différentes anatomies, et ceci, grâce à la coopération entre ces volets latéraux et des éléments rigides formant lien situés sur les bordures latérales de la serviette.

Ainsi, selon une de ses caractéristiques essentielles, la présente invention concerne un dispositif formant serviette périodique ou similaire, constitué d'une partie centrale comprenant un tampon absorbant de forme allongée à l'intérieur d'une enveloppe et des volets latéraux destinés à être rabattus par l'utilisatrice autour des bordures latérales de ladite partie centrale de la serviette ou autour des bords de l'entrejambe du sous-vêtement, caractérisé en ce qu'il comprend deux éléments formant lien chacun s'étendant sur au moins une partie de la longueur d'une bordure latérale de ladite partie centrale et étant fixé en outre, en au moins un point, au volet latéral situé du même côté, de manière à assurer la rétraction des bordures latérales lorsque lesdits volets latéraux sont rabattus.

Selon une caractéristique avantageuse de l'invention, chaque volet latéral présente un soufflet lorsque la partie centrale dudit dispositif est plane, ledit soufflet disparaissant lorsque les volets latéraux sont déployés et les bordures latérales sont rétractées.

Selon encore une autre caractéristique avantageuse de l'invention, les deux éléments formant lien sont distincts, chaque lien étant fixé en ses extrémités à une bordure latérale de ladite partie centrale de la serviette.

Par ailleurs, selon une variante de l'invention, les deux éléments précités forment un seul et même lien s'étendant sur toute la périphérie de ladite partie centrale.

Les éléments formant lien peuvent être constitués de tout matériau rigide, en particulier du type fil, cordonnet, ruban.

Selon une variante particulièrement avantageuse de l'invention, la serviette présente sur, au moins une partie de la longueur de ses bordures latérales, un bourrelet, en particulier un bourrelet tel qu'il est décrit dans le brevet européen EP 0520884, inclus dans la présente demande pour référence.

Dans cette variante, les éléments formant lien sont insérés à l'intérieur des bourrelets.

Les volets latéraux des serviettes périodiques de l'invention peuvent être aussi bien des volets de type rapporté, fixés sur la face de la serviette destinée à être en contact avec le sous-vêtement que des volets latéraux de type intégré, c'est-à-dire inclus dans la même enveloppe que la partie centrale de la serviette périodique.

Dans le cas où les volets latéraux sont rapportés sur la face de la serviette destinée à être en contact avec le sous-vêtement, une partie seulement des éléments formant lien se trouve à l'intérieur de l'enveloppe et des ouvertures sont ménagées dans l'enveloppe pour assurer le passage de ces éléments qui sont fixés sur la face supérieure des volets latéraux.

Dans le cas où les volets latéraux sont de type intégré, les éléments formant lien se trouvent sur toute leur longueur à l'intérieur de ladite enveloppe.

D'autres buts, caractéristiques et avantages apparaîtront clairement à la lumière de la description explicative qui va suivre et fait référence aux dessins annexés, représentant des modes de réalisation actuellement préférés de l'invention, donnés simplement à titre d'illustration, et qui ne sauraient donc en aucune façon limiter la portée de la présente invention.
- La figure 1 représente une vue en perspective d'un dispositif selon l'invention avant déploiement des volets latéraux ;
- La figure 2 représente une vue en coupe selon la ligne II-II de la figure 1.
- La figure 3 représente une vue en perspective du même dispositif incurvé par déploiement des volets latéraux.

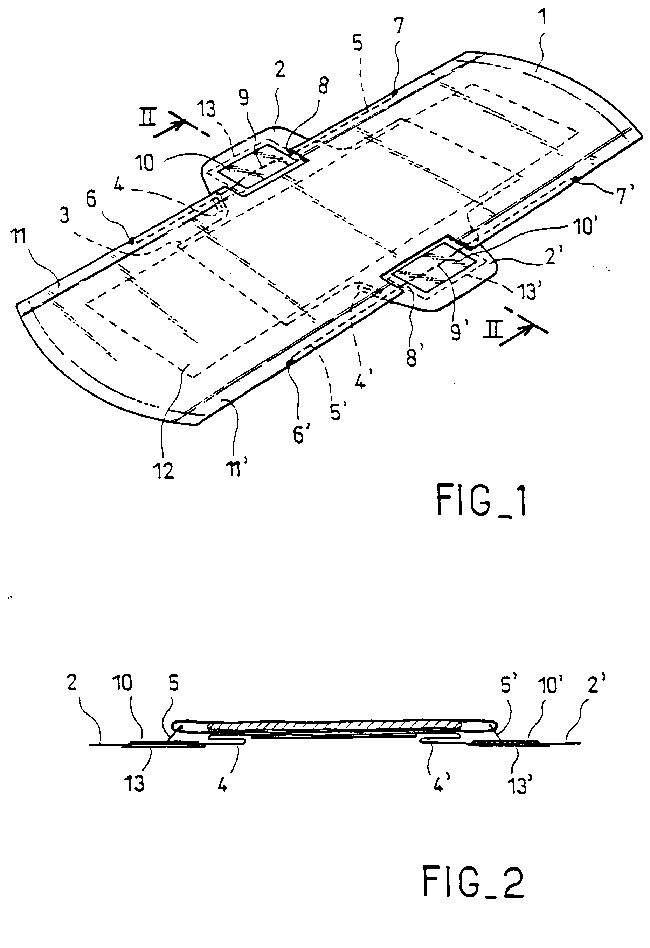

La description qui va suivre est faite en référence aux figures 1 à 3 qui correspondent au cas d'une serviette périodique selon l'invention dans laquelle les volets latéraux sont de type rapporté sur la face inférieure de la partie centrale.

La serviette selon l'invention représentée sur la figure 1 comprend classiquement un tampon de forme allongée à l'intérieur d'une enveloppe 1.

La face supérieure de cette enveloppe destinée à être en contact avec le corps est constituée d'une matière perméable aux fluides corporels.

Dans l'exemple de réalisation donnée à la figure 1, les volets latéraux 2, 2' sont rapportés en 3 sur la face inférieure de la partie centrale de la serviette périodique, comme cela apparaît sur les figures 1 et 2. Lorsque la partie centrale de la serviette est plane, les volets latéraux se trouvent partiellement repliés sous la partie centrale de la serviette formant un soufflet 4, 4'.

Deux cordonnets 5, 5' sont situés le long des bordures latérales de la partie centrale de la serviette et plus précisément de l'enveloppe 1. Ils sont fixés à ces bordures latérales en leurs extrémités 6, 7, 6', 7', par exemple en réalisant un noeud sur ces cordonnets à l'extérieur de l'enveloppe.

Les ouvertures 8, 8' situées sur les bordures latérales de la partie centrale de la serviette à la hauteur des volets latéraux permettent le passage de chacun des cordonnets qui est fixé de façon permanente en au moins un point 9, 9' de la face supérieure du volet latéral situé du même côté.

Dans le mode de réalisation représenté sur la figure 1, le collage du cordonnet sur le volet latéral est réalisé par l'intermédiaire d'un papier adhésif 10, 10'. Cette fixation du cordonnet peut être réalisée par tout moyen équivalent tel que thermosoudure, collage sans papier adhésif etc...

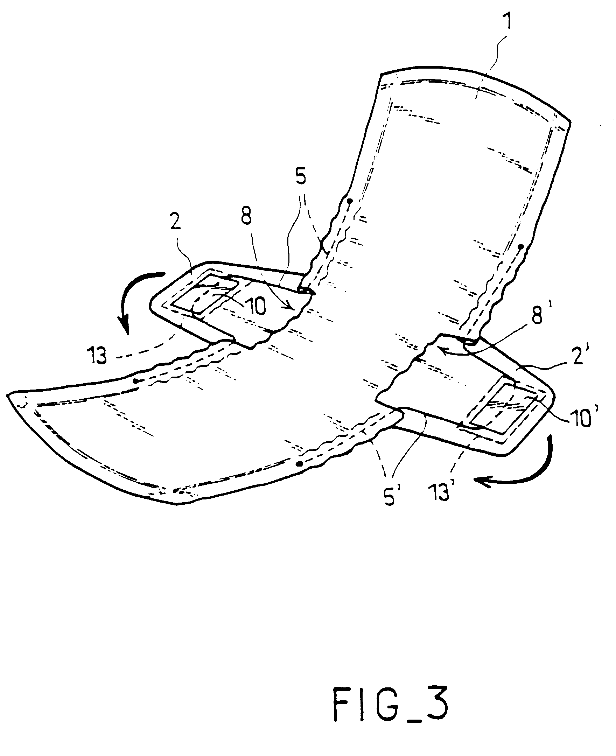

Le déploiement des volets latéraux , du fait de la présence des cordonnets, provoque simultanément la rétraction des bordures latérales de la serviette, en conséquence l'incurvation de la partie centrale de la serviette périodique comme cela apparaît sur la figure 3. Pour obtenir une incurvation symétrique de la partie centrale de la serviette, dans le sens longitudinal, les volets latéraux sont placés au centre des bordures latérales de la partie centrale de la serviette et les éléments formant lien rigide (ici cordonnets) sont fixés à ces volets latéraux en leur milieu.

Afin d'obtenir une incurvation plus accentuée à l'avant de la serviette, par exemple, la zone de fixation des éléments formant lien, sur les volets latéraux, serait décentrée dans le sens approprié.

On comprend, bien entendu, qu'il existe différentes positions intermédiaires entre celle représentée à la figure 1 et celle représentée à la figure 3 où les volets latéraux sont partiellement déployés correspondant à une disparition progressive des soufflets 4, 4'.

Selon une variante avantageuse de l'invention qui apparaît sur les exemples donnés dans les figures, les cordonnets sont situés à l'intérieur de bourrelets 11, 11' ménagés le long de la zone d'entrejambes et dont la fonction est d'améliorer le confort et l'efficacité de protection comme cela ressort de la demande de brevet européen EP 0520884.

Ce bourrelet peut être en particulier, comme illustré sur les figures 1 et 3, constitué par un retour, le long de chaque bord longitudinal de la couche supérieure de l'enveloppe, de la couche imperméable constituant la face inférieure de l'enveloppe. La couche inférieure de l'enveloppe forme dans ce cas un enveloppage en C de la couche perméable supérieure, correspondant au mode de réalisation du bourrelet représenté sur la figure 11 de la demande de brevet EP 0520884.

La face inférieure de la partie centrale de la serviette de l'invention, ainsi que la face inférieure ou supérieure des volets latéraux sont classiquement munies de tout moyen, en particulier adhésif, permettant leur fixation au linge de corps, les adhésifs étant eux-mêmes classiquement protégés avant utilisation par exemple par des bandes siliconées 12, 13, 13'.

On comprend aisément que les dimensions respectives des éléments constitutifs du dispositif puissent être modifiées de même que la nature des matériaux constitutifs de chacun de ces éléments.

En général, les différents éléments sont constitués des éléments classiques utilisés pour la fabrication de serviettes périodiques minces. Ainsi, l'élément tampon absorbant est de forme allongée, de préférence rectangulaire et est constitué d'un matériau cellulosique incluant une matière dite produit super-absorbant. De préférence, ce tampon est très mince de façon que l'épaisseur totale de la serviette n'excède pas 3 mm.

Dans une forme de réalisation préférée, on utilise un tampon sous la forme de matelas connu de l'homme du métier comme un matelas escargot.

On réalise ce type de matelas en déposant de la poudre super-absorbante sur la zone centrale d'une bande de matériau fibreux puis en rabattant les deux bords sur la partie centrale pour la recouvrir. On scelle le tout de manière que la poudre ne puisse migrer hors du matelas. On utilise, de préférence, pour le matériau fibreux un papier du type obtenu par voie sèche. Du fait de son mode de fabrication, ce type de matelas est de forme rectangulaire.

La face supérieure de l'enveloppe destinée à être en contact avec le corps est constituée de tout matériau dont le contact est suffisamment doux avec la peau et dont la structure et la texture se rapprochent de celles d'un tissu ; il peut s'agir, en particulier, d'une bande de matériau tissé ou non tissé, d'une bande de plastique perforée, d'un voile perforé.

Une couche imperméable constitue généralement la face inférieure de l'enveloppe ou lui est soudée ou collée. Cette couche est constituée d'une bande mince de matériau plastique imperméable tel que le polyéthylène éventuellement gaufré. Elle peut également être un complexe constitué d'un non-tissé composé de fibres liées, associé par exemple à un non-tissé du type "meltblown", composé de microfibres, ou bien ayant subi un traitement approprié de manière à présenter une certaine perméabilité à l'air où la vapeur tout en restant imperméable aux liquides dans les conditions habituelles d'utilisation. Tout autre produit présentant la propriété d'être perméable à l'air ou la vapeur d'eau tout en étant imperméable aux liquides corporels conviendrait également.

Les volets latéraux doivent avoir une flexibilité suffisante et sont, eux, constitués de tout matériau ou ensemble de matériaux pratiquement utilisé pour cette application; ils peuvent, en particulier, être constitués d'une simple feuille de matériau imperméable. Pour éviter une éventuelle irritation de la peau due aux frottements des éléments formant lien avec la peau au niveau des volets latéraux, plus particulièrement des volets de type rapporté, on prévoit un matériau recouvrant la surface de ces volets latéraux. Ce matériau est du type nontissé, voile de polyéthylène perforé ou tout autre matériau classiquement utilisé en surface des articles d'hygiène.

Bien entendu, les dispositifs selon l'invention sont munis sur leur face inférieure de tout moyen, en particulier adhésif, permettant la fixation de la serviette au linge de corps. Des moyens classiques de fixation des volets latéraux au linge de corps sont également prévus sur ou sous ceux-ci.

Des moyens de protection tels qu'une bande siliconée sont également prévus pour protéger les moyens de fixation mentionnés ci-dessus. Dans un mode de réalisation avantageux, on peut prévoir une bande siliconée qui, découpée, recouvre les adhésifs situés à la fois sous la partie centrale de la serviette et sous les volets latéraux. Ainsi lors de l'utilisation de la serviette, l'utilisatrice n'aurait qu'une seule bande siliconée à retirer, ce qui simplifierait l'utilisation de la serviette.

Dans un autre mode de réalisation de l'invention qui n'a pas été représenté dans les figures annexées, les deux cordonnets décrits dans le mode de réalisation précédent, sont remplacés par un seul cordonnet faisant le tour complet de la partie centrale de la serviette. Il est alors situé le long de toute la bordure formant la périphérie de l'enveloppe. Dans ce cas, aucun point de fixation des parties ou éléments formant ce lien n'est nécessaire. La tension exercée sur les volets latéraux par l'intermédiaire de ce lien aura pour effet non seulement de rétracter les bordures latérales de la serviette, en conséquence d'incurver la serviette dans le sens de la longueur, mais également d'incurver la serviette dans le sens transversal. Le dispositif formant serviette périodique prendra alors la forme d'une grande cuvette réceptacle et sera d'autant plus efficace.

L'avantage essentiel de l'invention telle qu'elle a été décrite précédemment est qu'elle permet à l'utilisatrice, en appliquant une tension plus ou moins importante sur les volets latéraux, de régler l'incurvation de la serviette selon son anatomie. Lors de la mise en place de la serviette sur le sous-vêtement, l'utilisatrice tire, en effet, sur les volets latéraux avant de les replier soit autour de la partie centrale de la serviette, soit autour du sous-vêtement. Cette traction a pour effet de tendre les éléments formant lien rigide qui provoquent l'incurvation de la serviette. Une fois les volets latéraux mis en place, la serviette garde sa forme. Dans un mode de réalisation particulier, les volets latéraux sont repliés autour des bordures latérales de la partie centrale de la serviette, avant leur mise en place sur la face supérieure du sous-vêtement. De ce fait, la forme rétractée ou incurvée de la serviette est déjà donnée avant sa mise en place sur le sous-vêtement.

## Revendications

1. Dispositif formant serviette périodique ou similaire constitué d'une partie centrale (8) comprenant un tampon absorbant de forme allongée à l'intérieur d'une enveloppe (1) et des volets latéraux (2, 2') destinés à être rabattus par l'utilisatrice autour des bordures latérales de la partie centrale de la serviette ou des bords d'entrejambes du sous-vêtement, caractérisé en ce qu'il comprend deux éléments rigides (5, 5') formant lien, chacun s'étendant sur au moins une partie de la longueur d'une bordure latérale de ladite partie centrale (8) et étant fixé, en au moins un point, au volet latéral situé du même côté, de manière à assurer la rétraction longitudinale des bordures latérales lorsque lesdits volets latéraux (2, 2') sont rabattus.

2. Dispositif selon la revendication 1, caractérisé en ce que chaque volet latéral présente un soufflet lorsque la partie centrale dudit dispositif est plane, ledit soufflet disparaissant lorsque lesdits volets latéraux sont déployés et les bordures latérales sont rétractées.

3. Dispositif selon la revendication 1 ou 2, caractérisé en ce que les deux éléments (5, 5') formant lien sont distincts et en ce que chaque lien (5, 5') est fixé en ses extrémités (6, 7, 6', 7') à une bordure latérale de ladite partie centrale (8).

4. Dispositif selon la revendication 1 ou 2, caractérisé en ce que les deux éléments précités forment un seul et même lien s'étendant sur toute la périphérie de ladite partie centrale (8).

5. Dispositif selon l'une des revendications précédentes, caractérisé en ce que ledit élément rigide formant lien est un élément du type fil, cordonnet ou ruban.

6. Dispositif selon l'une des revendications précédentes, caractérisé en ce que le dispositif présente, sur au moins une partie de la zone d'entrejambes, un bourrelet (11, 11') à l'intérieur duquel sont insérés les deux éléments précités formant lien.

7. Dispositif selon l'une des revendications précédentes, caractérisé en ce que les volets latéraux sont des volets rapportés fixés sur la face dudit dispositif destiné à être en contact avec le sous-vêtement.

8. Dispositif selon la revendication 7, caractérisé en ce que ladite enveloppe présente des ouvertures (8, 8') situées sur lesdites bordures latérales permettant le passage desdits éléments formant lien et en ce que lesdits éléments formant lien sont fixés sur la face supérieure desdits volets latéraux.

## Claims

1. Device forming a sanitary towel or the like consisting of a central part (8) comprising an absorbent tampon with an elongated shape inside an envelope (1) and side flaps (2, 2') intended to be turned back by the user around the side edges of the central part of the towel or of the edges of the crotch of the undergarment, characterized in that it includes two rigid elements (5, 5') forming a tie, each extending over at least part of the length of a side edge of the said central part (8) and being attached, at least at one point, to the side flap situated on the same side, so as to ensure the longitudinal retraction of the side edges when the said side flaps (2, 2') are turned back.

2. Device according to claim 1, characterized in that each side flap has a gusset when the central part of the said device is flat, the said gusset disappearing when the said side flaps are deployed and the side edges are retracted.

3. Device according to claim 1 or 2, characterized in that the two elements (5, 5') forming a tie are distinct and in that each tie (5, 5,) is attached at its ends (6, 7, 6', 7') to a side edge of the said central part (8).

4. Device according to claim 1 or 2, characterized in that the aforementioned two elements form one and the same tie extending over all the periphery of the said central part (8).

5. Device according to any of the preceding claims, characterized in that the said rigid element forming a tie is an element of the thread, string or ribbon type.

6. Device according to any of the preceding claims, characterized in that the present device has a cushion (11, 11') on at least part of the crotch area, inside which the two aforementioned elements forming a tie are inserted.

7. Device according to any of the preceding claims, characterized in that side flaps are separate flaps attached to the face of the said device intended to be in contact with the undergarment.

8. Device according to claim 7, characterized in that the said envelope has openings (8, 8') situated on the said side edges, permitting the passage of the said elements forming a tie and in that the said elements forming a tie are attached to the upper face of the side flaps.

## Patentansprüche

1. Vorrichtung, die eine Damenbinde oder ähnliches bildet, gebildet von einem Zentralteil (8), welches ein absorbierendes Saugelement von länglicher Form im Inneren einer Umhüllung (1) und zwei seitliche Flügel (2, 2') aufweist, die bestimmt sind, von der Benutzerin um seitliche Ränder des Zentralteils der Binde oder von im Schritt befindlichen Rändern der Unterwäsche geklappt zu werden, dadurch gekennzeichnet, daß sie zwei unelastische Elemente (5, 5') aufweist, die eine Verbindung bilden, von denen sich jedes auf zumindest einem Teil der Länge eines seitlichen Randes des Zentralabschnittes (8) erstreckt und in zumindest einem Punkt an dem auf derselben Seite angeordneten Flügel fixiert ist, so daß das Zusammenziehen der Seitenränder in Längsrichtung sichergestellt ist, wenn die seitlichen Flügel (2, 2')umgeklappt sind.

2. Vorrichtung gemäß Anspruch 1, dadurch gekennzeichnet, daß jeder seitliche Flügel eine Faltung zeigt, wenn der Zentralteil der Vorrichtung eben ist, wobei die Faltung verschwindet, wenn die seitlichen Flügel ausgebreitet und die seitlichen Ränder zurückgezogen sind.

3. Vorrichtung gemäß Anspruch 1 oder 2, dadurch gekennzeichnet, daß die beiden Elemente (5, 5'), die eine Verbindung bilden, verschieden sind und daß jede Verbindung (5, 5') an ihren Enden (6, 7, 6', 7') an einem seitlichen Rand des Zentralteils (8) fixiert ist.

4. Vorrichtung gemäß Anspruch 1 oder 2, dadurch gekennzeichnet, daß die beiden vorgenannten Elemente ein und dieselbe Verbindung bilden, welche sich über die gesamte Peripherie des Zentralteiles (8) erstreckt.

5. Vorrichtung gemäß einem der vorstehenden Ansprüche, dadurch gekennzeichnet, daß das unelastische Element, welches eine Verbindung bildet, ein Faden, eine dünne Schnur oder ein Band ist.

6. Vorrichtung gemäß einem der vorstehenden Ansprüche, dadurch gekennzeichnet, daß die Vorrichtung an zumindest einem Teil ihrer Schrittzone einen Wulst (11, 11') aufweist, in dessen Inneres die beiden vorgenannten Elemente eingesetzt sind, welche die Verbindung bilden.

7. Vorrichtung gemäß einem der vorstehenden Ansprüche, dadurch gekennzeichnet, daß die seitlichen Flügel angesetzte Flügel sind, die auf der Seite der Vorrichtung befestigt sind, die dazu bestimmt ist, in Kontakt mit der Unterwäsche zu kommen.

8. Vorrichtung gemäß Anspruch 7, dadurch gekennzeichnet, daß die Umhüllung Öffnungen (8, 8') zeigt, die auf den seitlichen Rändern angeordnet sind, welche den Durchgang der Elemente ermöglichen, welche die Verbindung bilden, und daß die die Verbindung bildenden Elemente auf der oberen Seite der seitlichen Flügel befestigt sind.
